# EUROPEAN PATENT SPECIFICATION

(11) **EP 2 371 266 B1**
(45) Date of publication and mention of the grant of the patent: **13.03.2019**
(21) Application number: 11152573.9
(22) Date of filing: 28.01.2011
(51) Int. Cl.: A61B 1/005, A61B 1/00, G02B 23/24

(54) **Flexible tube for endoscope and method for producing the same**
Flexible Röhre für Endoskop und Herstellungsverfahren dafür
Tube flexible pour endoscope et son procédé de fabrication

(30) Priority: 31.03.2010 JP 2010084551
(43) Date of publication of application: 05.10.2011
(73) Proprietor: FUJIFILM Corporation, Tokyo (JP)
(72) Inventor: NAKAMURA, Shigeru, Kanagawa (JP)
(74) Representative: Klunker IP Patentanwälte PartG mbB

(56) References cited:
- JP-A- 2005 245 517
- JP-A- 2006 061 205
- JP-A- 2006 061 205
- US-A- 3 235 537
- US-A- 3 235 537
- US-A1- 2002 028 984
- US-A1- 2002 028 984
- US-A1- 2009 069 631

## Description

### BACKGROUND

### Field of the Invention

The present invention relates to an endoscope comprising a flexible tube and a method for producing the tube.

### Related Art

Medical endoscopes perform an observation of organs and the like by having the insertion unit inserted into a body cavity, or various treatments or remedies by using a treatment tool inserted into a treatment tool insert-through channel of the endoscope. Therefore, when an endoscope that has once been used is to be reused in another patient, it is required to disinfect and sterilize the endoscope after the completion of examination and treatment, in order to prevent the infection between patients mediated by the endoscope. Disinfection or sterilization is achieved by methods of using a disinfectant liquid, ethylene oxide gas, formalin gas, hydrogen peroxide gas plasma, ozone, a sterilized autoclave which uses high temperature high pressure steam, and the like.

A hydrogen peroxide plasma method is a method of decomposing hydrogen peroxide by means of plasma to generate active hydroxy radicals, and thereby achieving sterilization.

Furthermore, a disinfection and sterilization method that has been widely popularized involves the use of an autoclave which sterilizes an endoscope with high temperature high pressure vapor. This method has numerous merits such as high reliability in the sterilization effect, lack of residual toxicity, and low running cost.

For all of these sterilization methods, the medical instruments are subjected to significant damage in such an environment. In addition, there has been traditionally a problem that the polyurethane elastomer used in the protection of the flexible tube in the insertion unit of a soft endoscope, has the surface gradually attacked by oxidation during a hydrogen peroxide plasma treatment, and is also easily hydrolyzed by an autoclave treatment, so that the polyurethane elastomer loses its function of protecting the interior of the flexible tube.

For this reason, there have been made various attempts in connection with the casing layer material having high durability against autoclave sterilization treatment.

For example, there have been disclosed a flexible tube having a casing layer formed of an olefin-based thermoplastic elastomer and a polyolefin resin (see, for example, Japanese Patent Application Laid-Open (JP-A) No. 2005-21243), and a flexible tube having a casing made of a styrene-based elastomer (see, for example, JP-A No. 2005-224538). Furthermore, it is disclosed that a flexible tube having improved adhesion may be obtained by using an olefin-based thermoplastic elastomer for the casing, and applying a reactive crosslinking type fluorine coating material above the casing, with a primer formed of a chlorinated olefin interposed therebetween (see, for example, JP-A No. 2006-314521).

The reactive crosslinking type fluorine coating material used herein may give an ameliorating effect to some extent. However, since the coating material utilizes the formation of a urethane bond as a result of reactive curing, this bonding group is cleaved by hot steam during autoclaving, and as a result, practically sufficient durability is not obtainable with this fluorine coating material.

JP 2006-061205 A and US 2002/028984 A1
each disclose an endoscope comprising an insertion section which includes a flexible tube, wherein the flexible tube comprises a metallic core material which has a helical tube formed by winding a band-shaped member in a helical form, and a reticulated tube formed along an outer periphery of the helical tube; a casing layer which is formed on a surface of the metallic core material, wherein the casing layer has an inner surface adhering to the surface of the core material to obtain an integrated body; an adhesiveness enhancing layer which is formed along an outer periphery of the casing layer; and an overcoat layer which is formed along an outer periphery of the adhesiveness enhancing layer and contains a fluororesin.

### SUMMARY

The present invention has been made in view of the above circumstances and provides an endoscope as claimed in claim 1, and
a method for producing a flexible tube of the endoscope as claimed in claim 3.

### BRIEF DESCRIPTION OF THE DRAWINGS

Exemplary embodiments of the present invention will be described in detail, based on the following figures, wherein:
FIG. 1 is a schematic configuration diagram for an endoscope according to a present exemplary embodiment; and
FIG. 2 is a cross-sectional view showing an example of the constitution of the flexible tube of an endoscope according to an exemplary embodiment.

### DETAILED DESCRIPTION OF THE INVENTION

Hereinafter, an exemplary embodiment according to the present invention will be described based on the drawings.

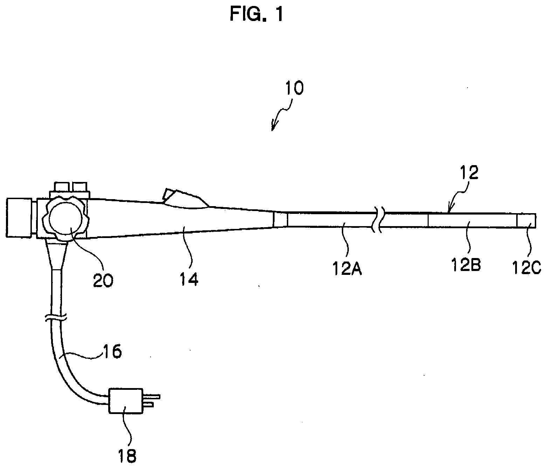

First, the overall configuration of an endoscope 10 according to the present exemplary embodiment will be explained. FIG. 1 is a schematic configuration diagram showing the overall configuration of the endoscope 10 according to the exemplary embodiment.

The endoscope 10 according to the exemplary embodiment includes, as shown in FIG. 1, a long-shaped insertion unit 12 which is inserted into a body cavity of a patient, and a main body operation unit 14 is linked to a base end section of the insertion unit 12. This main body operation unit 14 is connected with a long-shaped light guide soft section 16 which is connected detachably from and attachably to a light source apparatus (not depicted), At the front tip area of the light guide soft section 16, a connection section 18 having a terminal that is connected to the light source apparatus (not depicted) is provided. Furthermore, the main body operation unit 14 is provided with an operation knob 20 for operating the insertion unit 12.

The insertion unit 12 includes a flexible tube 12A which constitutes a major portion of the length area in the longitudinal direction (axial direction) from the area linked to the main body operation unit 14; an angle section 12B which is linked to the front tip side in the longitudinal direction of this flexible tube 12A; and a front tip section body 12C which is linked to the front tip side in the longitudinal direction of the angle section 12B and is also mounted inside with an objective optical system or the like.

The angle section 12B is arranged to be bent by remote operation by rotating the operation knob 20 provided at the insertion unit 12. Furthermore, the light guide soft section 16 is also arranged to have an almost the same structure as that of the flexible tube 12A of the insertion unit 12.

The flexible tube 12A has a length secured, which allows the front tip section body 12C to reach to the inside of a certain subject area of observation, and the flexible tube 12A is set to have a length that is capable of separating the main body operation unit 14 from the patient or the like to the extent that the operator is not disrupted when the operator grabs and operates the main body operation unit. The flexible tube 12A is required to have flexibility over almost the entire length of the tube, and particularly the part that is inserted into a body cavity of a patient is made to have a more highly flexible structure.

Furthermore, the flexible tube 12A needs to have certain rigidity against bending particularly at the area linked to the main body operation unit 14, in order to obtain a push driving force when the flexible tube is inserted into a body cavity or the like. It is also preferable that the flexible tube 12A have higher flexibility particularly at the area linked to the angle section 12B, so that when the angle section 12B is curved, the flexible tube is allowed to conform to this curved shape to a certain extent.

The flexible tube 12A has a light guide, an image guide (a signal cable in the case of an electronic endoscope), a treatment tool insert-through channel, an air/water transport tube and the like (all not depicted) mounted inside the tube-shaped section.

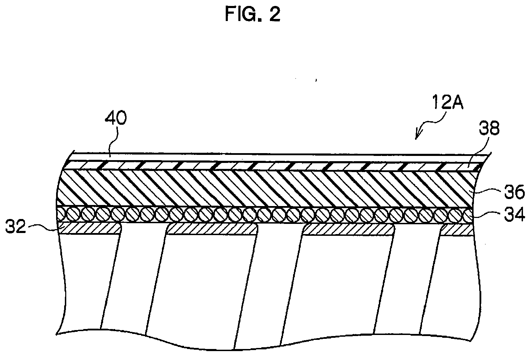

FIG. 2 is a cross-sectional view showing an example of the flexible tube 12A according to an exemplary embodiment.

As shown in FIG. 2, the flexible tube 12A of the invention has a casing layer 36 containing a thermoplastic elastomer that will be described in detail below, an adhesiveness enhancing layer 38 and an overcoat layer 40, on the surface of a metallic core material which has a helical tube 32 formed by winding a band-shaped member in a helical form, and along an outer periphery of the helical tube, a reticulated tube 34 formed into a ring shape by braiding fine wires.

Hereinafter, the respective layers that constitute the flexible tube according to the exemplary embodiment and the materials constituting the respective layers, together with the method for production thereof, will be explained in detail.

### (Casing layer)

First, the casing layer 36 will be explained.

The casing layer 36 in the flexible tube for an endoscope according to the invention contains an olefin-based elastomer or a styrene-based elastomer. The thermoplastic elastomer used in the invention is a thermoplastic elastomer composed of saturated hydrocarbons, and refers to a product formed from a polymer which does not contain a bond group that is susceptible to decomposition, such as a urethane bond or an ester bond, in the polymer and is composed of carbon chains.

More specifically, the thermoplastic elastomer may be an olefin-based thermoplastic elastomer or a styrene-based thermoplastic elastomer.

Examples of the olefin-based elastomer include elastomers of various forms such as blend type, dynamic crosslinked type and polymerized type, and among these, an elastomer having a hardness that is suitable as the casing for an endoscope may be selected and used.

The olefin-based elastomer that may be used in the exemplary embodiment is also available as commercially marketed products, and specific examples thereof include SANTOPRENE (trade name, manufactured by Exxon Mobil Corp.), SARLINK (trade name, manufactured by Toyobo Co., Ltd.), and ZELAS (trade name, manufactured by Mitsubishi Chemical Corp.). Among them, ZELAS, which has a polymerized type fine structure, is suitable for forming uniform thin-walled tubes and is particularly preferable as the material for casing layer.

As another example of the casing formed of saturated hydrocarbons according to the invention, a styrene-based thermoplastic elastomer is also preferably used. A styrene-based elastomer is a block copolymer having a styrene unit, which is composed of hard segments and soft segments.

The styrene-based elastomer is also available as commercially marketed products, and specific examples thereof include RABALON (trade name, manufactured by Mitsubishi Chemical Corp.), and SEPTON (trade name, manufactured by Kuraray Co., Ltd.). The styrene-based elastomer may be arbitrarily selected from these commercially available products and used.

With regard to the thermoplastic elastomer used in the formation of the casing layer according to the invention, use is made of a thermoplastic elastomer which does not melt at the heating temperature during an autoclave treatment, or even though not melting, does not cause noticeable creep.

The casing layer 36 according to the invention may contain various additives in addition to the thermoplastic elastomer.

For example, if it is wished to impart a light blocking property to the casing layer 36, the casing layer may contain a black pigment or the like.

The casing layer is preferably colored black so as to prevent scattering of the illumination light inside the body cavity and any undesirable leakage of light from the endoscope. Examples of the black pigment that may be used for black coloration include carbon black, titanium black, a ferrosoferric-based black pigment, and organic black pigments. Furthermore, this black pigment may be a pigment composition containing multiple components having a function of turning white upon irradiation with laser light.

From the viewpoint of the light blocking property, the content of the black pigment contained in the casing layer is preferably in the range of 0.5% by mass to 5% by mass relative to the total mass of the composition constituting the casing layer, and the content is more preferably in the range of 1% by mass to 3% by mass.

The composition for the formation of a casing layer which is used for forming a casing layer, is prepared by thoroughly mixing a thermoplastic elastomer and a black pigment.

In regard to the method for producing a casing layer, a tube for hollow casing layer may be formed by using a tube molding machine, or a casing layer may be formed directly on a metallic core material by using coating-molding with an extrusion molding machine, and the like. In the case of the former method, the tube is subjected to a processing of covering a metallic core material with the tube, after molding.

### (Enhancement of adhesiveness between metallic core material and casing layer)

When an integrated body is formed by covering a metallic core material with the casing layer obtained as described above, the adhesiveness between the casing layer and the metallic core material may be enhanced by applying a primer on the surface of the metallic core material, and then coating-molding the casing layer or covering the primer-coated core material with the casing layer.

Examples of the primer include a silane coupling agent, a titanate-based coupling agent, and a zirconate-based coupling agent, and it is preferable to use a silane coupling agent.

The silane coupling agent is preferably methoxysilane having a vinyl group, or ethoxysilane having a vinyl group. Specific examples include vinyltrimethoxysilane and vinyltriethoxysilane.

In regard to the method for adhesion, when a dilute solution of a silane coupling agent is applied and dried on the surface of a metallic core material, the coating is subjected to a heating treatment as necessary, and then the metallic core material is adhered with a casing layer and heated in a subsequent step, the casing layer and the metallic core material undergo vulcanizing adhesion, and thus an integrated body of a casing layer and a metallic core material is obtained.

In the case of forming a casing layer directly on a metallic core material using a coating-molding machine, a metallic core material which has been treated in advance with a silane coupling agent may be used.

The casing layer is adhered to the surface of the metallic core material to obtain an integrated body, such that the inner surface of the casing layer (the surface in contact with the core material) is firmly adhered only at the area that is brought into contact with the reticulated tube, which is the outermost layer of the metallic core material, and thereby a flexible tube excellent in flexibility and durability is obtained, in which the satisfactory flexibility of the reticulated tube is coupled with the high elasticity of the casing layer containing a thermoplastic elastomer as a main component.

According to the invention, the thickness of the casing layer is appropriately selected based on the diameter of the flexible tube for an endoscope or the use. From the viewpoint of the metallic core material protective property and durability, the thickness is preferably at least about 100 µm or greater, and from the viewpoint of not impairing the flexibility, the thickness is preferably 2000 µm or less.

### (Atmospheric pressure plasma treatment)

The surface of the casing layer 36 of the flexible tube 12A is subjected to an atmospheric pressure plasma treatment, prior to the formation of an adhesiveness enhancing layer 38 which is disposed along an outer periphery of the casing layer. As a result of the atmospheric pressure plasma treatment, the adhesive strength between the casing layer 36 and the adhesiveness enhancing layer 38 is enhanced.

The apparatus used in the atmospheric pressure plasma treatment may be selected from any commercially available atmospheric pressure plasma apparatuses such as those manufactured by Plasmatreat GmbH and those manufactured by Kasuga Electric Works, Ltd. The plasma treatment conditions may be appropriately adjusted and used in accordance with the capacity of the apparatus, but typically, it is preferable to set the treatment conditions such that for a casing layer 36 formed from a thermoplastic elastomer having a contact angle of water of about 70° to 90°, the contact angle of water at the surface of the casing layer 36 is from 15° to 60°.

The atmospheric pressure plasma treatment on the surface of the casing layer 36 is usually carried out after the casing layer 36 is laminated on the metallic core material, from the viewpoint of workability.

### (Adhesiveness enhancing layer)

The adhesiveness enhancing layer 38 formed on an outer periphery of the casing layer 36 is formed to include a flexible epoxy resin, and the flexible epoxy resin is preferably an epoxy resin which is a reaction product of a liquid epoxy resin main agent and an amine-based curing agent.

In order to obtain a flexible epoxy resin for the formation of the adhesiveness enhancing layer 38, a method of allowing an epoxy main agent having a flexible structure and a curing agent having a flexible structure to react, may be used.

The epoxy main agent having a flexible structure may be a compound having a structure in which epoxy groups are present at both ends and the epoxy groups are bonded via a linking group having a flexible structure. Specific examples include butanediol diglycidyl ether, a reaction product of polypropylene glycol and epichlorohydrin (specifically, for example, DER 732 (trade name) manufactured by Dow Chemical Company), and an epoxy compound having a bisphenol type epoxy linked via a flexible skeleton (specifically, for example, EXA-4850 (trade name) manufactured by DIC Corp.).

The curing agent having a flexible structure is preferably a compound which has nucleophilic reactive groups at the terminals and has these groups linked via a linking group having a flexible structure, and more specifically a compound which has amino groups at the terminals and has these amino groups linked via a linking group having a flexible structure. The linking group may have more amino groups in the linking chain at sites other than the terminals, or may be such that the linking chain has branch chains, and the branch chains have amino groups.

Specific examples of the curing agent having a flexible structure include polyetheramines. Specific preferred examples of a polyetheramine that is suitable for the invention include commercially marketed products such as JEFFAMINE D400, D2000, XJT-542 and T-403 (all trade names, manufactured by Huntsman International LLC).

Upon the production of a flexible epoxy resin, it is preferable to allowing the epoxy groups carried by the epoxy main agent and the amino groups of the curing agent that react with those epoxy groups, to react in their equivalent amounts.

There are no particular limitations on the method for producing the adhesiveness enhancing layer 38, and the adhesiveness enhancing layer may be provided by dissolving a flexible epoxy resin composition containing an epoxy main agent having a flexible structure and a curing agent having a flexible structure, in a solvent, applying the composition on the surface of the casing layer 36 which has been atmospheric pressure plasma treated, and drying the composition, or may be provided by immersing an integrated body of a metallic core material and a casing layer in a solution of a flexible epoxy resin composition, and then pulling out and drying the integrated body. Here, there are no particular limitations on the solvent used to dissolve the flexible epoxy resin, as long as the solvent is capable of dissolving the epoxy resin composition to be used, at a desired resin concentration.

The thickness of the adhesiveness enhancing layer is preferably in the range of 0.1 µm to 10 µm, and more preferably in the range of 0.5 µm to 5 µm.

### (Overcoat layer)

The flexible tube 12A of the invention further has an overcoat layer 40 on the surface of the adhesiveness enhancing layer 38 which is provided on an outer periphery of the casing layer 36, for the purpose of enhancing durability.

As the resin that forms the overcoat layer 40, a fluororesin having a vinylidene fluoride unit is used. The resin is preferably a soft fluororesin having higher hardness than the thermoplastic elastomer used in the casing layer, and from the viewpoint of the ease of formation of the overcoat layer, a solvent-soluble soft fluororesin is preferred compare to a reactive curing type fluororesin.

Although the boundary between the thermoplastic elastomer and the soft fluororesin is not clearly defined, but according to the invention, a resin having a hardness of approximately A70 or less in terms of Shore hardness is called a fluorine-based elastomer, and a resin having a hardness region measurable in terms of Shore D hardness, which exceeds A80, is called a soft fluororesin.

The soft fluororesin used in the formation of an overcoat layer according to the invention preferably has a Shore hardness of from 40 to 60, and more preferably has a Shore hardness of from 44 to 55.

The fluororesin having a vinylidene fluoride unit (hereinafter, appropriately referred to as particular fluororesin) is preferably a soft fluororesin having a hardness such as described above, and may be selected from copolymers of a monomer component which results in a crystalline polymer, and a non-crystalline polymer component.

There are no particular limitations on the particular fluororesin as long as the fluororesin has a vinylidene fluoride unit as a polymerization component, and from the viewpoint of the effects of the invention, includes the vinylidene fluoride unit at a proportion of 40% by mole to 90% by mole.

When the content ratio of the vinylidene fluoride unit is in the range described above, a uniform overcoat layer is formed, and a sufficient adhesive power with the adjacent adhesiveness enhancing layer containing a soft epoxy resin is obtained. If the content ratio of the vinylidene fluoride unit exceeds 90% by mole, it tends to be difficult to form a uniform polymer layer. Thus, from this point of view, a copolymer including a unit other than the vinylidene fluoride unit for an enhancement of coatability may also be regarded as a preferred embodiment.

From the viewpoint of enhancing the oxidation resistance of the particular fluororesin and the liquid component penetration suppressive capability, it is preferable that the particular fluororesin include a fluorine-containing unit having a fluoroalkyl group, a fluorovinyl group or the like as a copolymerization component.

A preferred example may be a polymer constituted to include a vinylidene fluoride unit at a proportion of 40% by mole to 90% by mole and further include either a fluoroalkylene group-containing unit at a proportion of 10% by mole to 50% by mole or a fluorovinyl ether unit at a proportion of 10% by mole to 50% by mole; or a polymer constituted to include a vinylidene fluoride unit at a proportion of 40% by mole to 90% by mole, a fluoroalkylene group-containing unit at a proportion of 10% to 30% by mole and a fluorovinyl ether unit at a proportion of 10% by mole to 30% by mole.

Examples of the fluoroalkylene group-containing unit include hexafluoropropylene and trifluoroethylene.

Examples of the fluorovinyl ether unit include perfluorovinyl ether.

Specific examples of the particular fluororesin that is used in the formation of the overcoat layer according to the invention, include a tercopolymer of tetrafluoroethylene/hexafluoropropylene/vinylidene fluoride. This has the same composition as fluororubbers, but by controlling the copolymerization ratio, the fluororesin may be produced into a resin having appropriate flexibility, unlike the copolymers in the rubbery phase region, and such a resin may be suitably used. The composition region capable of forming a soft resin is described in US Patent No. 3,235,537, and the soft resins described in this document may be used in the invention as well.

Other preferred examples of the particular fluororesin include resins obtained by graft polymerizing a crystalline polymer to a non-crystalline polymer. Examples of the soft fluororesin having a graft chain include graft polymers obtained by grafting a homopolymer of vinylidene fluoride as a branch polymer to a stem polymer of vinylidene fluoride/ hexafluoropropylene or vinylidene fluoride/chlorotrifluoroethylene.

The particular fluororesin used in the overcoat layer is also available as commercially marketed products, and examples of the former tercopolymer type solvent-soluble soft resin include THV220A (trade name, manufactured by Sumitomo 3M, Ltd.), while examples of the latter soft fluororesin having a graft chain include CEFRAL SOFT G120, G150 and G180 (trade name, manufactured by Central Glass Co., Ltd.). Among them, CEFRAL SOFT is preferred from the viewpoint of strength and heat resistance.

There are no particular limitations on the method for producing an overcoat layer, but examples include a method of dissolving a particular fluororesin such as described above in a solvent, and applying and drying the solution.

There are no particular limitations on the solvent that is used for dissolving the particular fluororesin when a coating liquid is prepared, as long as the solvent is capable of dissolving the particular fluororesin to be used at a desired resin concentration. Examples of the solvent include amide-based solvents such as dimethylformamide, N-methylformamide and N-methylpyrrolidone; carbonyl-based solvents such as methyl ethyl ketone and cyclohexanone; and ester-based solvents such as butyl acetate and ethyl acetate. Among these, the solvent may be appropriately selected in consideration of the solubility of the particular fluororesin, dryability and the like.

The concentration of the particular fluororesin in the coating liquid for the formation of an overcoat layer is preferably in the range of from 1% by mass to 10% by mass relative to the mass of the total solid contents.

Furthermore, according to the invention, the most preferably used polymer in which a homopolymer of vinylidene fluoride is grafted to a main chain portion of vinylidene fluoride/chlorotrifluoroethylene (for example, CEFRAL SOFT manufactured by Central Glass Co., Ltd.) is sparingly soluble in conventional solvents, and therefore, it is preferable to use cyclohexanone as the solvent. When cyclohexanone is used and the solution is adjusted to a warmed state, dissolution at a high concentration may be achieved, and there is obtained an unusual property that the particular fluororesin gels without precipitating when cooled.

Therefore, in the case of using the particular graft copolymer as the particular fluororesin, when cyclohexanone is used as the solvent and the property of gelling is utilized, an overcoat layer having a uniform thickness may be formed, without the occurrence of cloudiness of the film during the film formation by solution coating, and without the problem of liquid dripping during drying.

It is more preferable that the coating liquid for the formation of an overcoat layer contain a co-crosslinking agent containing plural unsaturated bonds, in addition to the soft fluororesin. When a co-crosslinking agent is incorporated, and then energy is applied by heating, irradiation with radiation or the like, a crosslinked structure is formed, and the heat resistance of the overcoat layer is enhanced. In addition to that, the adhesive strength between the overcoat layer and the adhesiveness enhancing layer and between the adhesiveness enhancing layer and the casing layer is enhanced, and a flexible tube having more excellent physical durability and thermal durability may be obtained. Here, a polyfunctional vinyl compound or the like may be used as the co-crosslinking agent, and specific examples thereof include triallyl isocyanurate and trimethacryl isocyanurate.

Examples of the method of applying an overcoat layer include dip coating, spray coating, and a slit coating method by which a liquid is ejected on a transparent casing layer through the slits of a coating head. Among these, a dip coating method is suitable from the viewpoint that a film of overcoat layer having a predetermined small thickness may be formed.

The thickness of the overcoat layer is required to be a thickness sufficient for protecting the casing layer from damages due to wear-out. On the other hand, if the overcoat layer is excessively thick, the flexibility of the casing is impaired. Therefore, the thickness is preferably in the range of from 1 µm to 100 µm, and more preferably in the range of from 2 µm to 30 µm.

Exemplary embodiments according to the invention will be listed hereinafter.
<1> An endoscope comprising an insertion section which includes a flexible tube, wherein the flexible tube comprises:
   a metallic core material which has a helical tube formed by winding a band-shaped member in a helical form, and a reticulated tube formed along an outer periphery of the helical tube;
   a casing layer which is formed on a surface of the metallic core material, wherein the casing layer has an inner surface adhering to the surface of the core material to obtain an integrated body, and further wherein the casing layer has a surface at an outer periphery thereof, that is subjected to an atmospheric pressure plasma treatment, and contains an olefin-based elastomer or a styrene-based elastomer;
   an adhesiveness enhancing layer which is formed along an outer periphery of the casing layer and contains a flexible (soft) epoxy resin; and
   an overcoat layer which is formed along an outer periphery of the adhesiveness enhancing layer and contains a fluororesin, wherein the fluororesin is a solvent-soluble fluororesin having a vinylidene fluoride unit as a polymerization component at a proportion of from 40% by mole to 90% by mole, and the inner surface of the casing layer is adhered to the metallic core material only at the area in contact with the reticulated tube.
<2> The flexible tube for an endoscope of <1>, wherein the flexible (soft) epoxy resin is a reaction product between a curing agent having an amino group and an epoxy resin main agent selected from butanediol diglycidyl ether, a reaction product of polypropylene glycol and epichlorohydrin, or a bisphenol epoxy compound.
<3> A method for producing a flexible tube of an insertion section of an endoscope as indicated under <1> above, the method comprising:
   coating an olefin-based elastomer or a styrene-based elastomer on a metallic core material having a helical tube formed by winding a band-shaped member in a helical form and a reticulated tube formed along an outer periphery of the helical tube, and thereby forming a casing layer on the metallic core material, wherein an inner surface of the casing layer is adhered to the surface of the core material to obtain an integrated body;
   subjecting a surface at an outer periphery of the casing layer to an atmospheric pressure plasma treatment to adjust a water contact angle at the surface of the casing layer to a value of from 15° to 60°;
   applying a coating liquid composition containing a flexible (soft) epoxy resin along an outer periphery of the atmospheric pressure plasma-treated casing layer, and heating and drying the coating liquid composition, to form an adhesiveness enhancing layer; and
   applying a coating liquid containing a fluororesin having a vinylidene fluoride unit as a polymerization component at a proportion of from 40% by mole to 90% by mole onto a surface of the adhesiveness enhancing layer, and drying and curing the coating liquid, to form an overcoat layer,
   wherein the inner surface of the casing layer is adhered to the metallic core material only at the area in contact with the reticulated tube.

### EXAMPLES

Hereinafter, the invention will be specifically described by way of Examples, but the invention is not intended to be limited to the Examples, and various modifications, alterations and improvements may be made.

### [Example 1]

### (Formation of casing layer)

A metallic core material having an outer diameter of 5.0 mm and a length of 550 mm, which was produced using SUS304 and was composed of a helical tube 32 and a reticulated tube 34, was produced.

A composition for the formation of a casing layer prepared by adding 2% by mass of a masterbatch for black coloration to ZELAS MC707(trade name, manufactured by Mitsubishi Chemical Corp.), which is an olefin-based thermoplastic elastomer, was used, an extrusion molding machine for resin was used, and extrusion was performed at a head temperature of 200°C, to produce a tube-shaped casing layer 36 having a thickness of 0.3 mm.

The metallic core material was covered with the tube produced as described above, and thus an integrated body was formed.

Subsequently, the surface of this integrated body was subjected to a plasma treatment using an atmospheric pressure plasma apparatus (manufactured by Kasuga Electric Works, Ltd.), and thereby surface modification was performed. Due to this atmospheric pressure plasma treatment, the water contact angle on the surface of the casing layer 36 was changed from 85° to 40°.

### (Formation of adhesiveness enhancing layer)

Subsequently, a coating liquid composition for the formation of an adhesiveness enhancing layer having a liquid state epoxy resin and a curing agent, which had the following composition, was applied on the coating tube at the surface of the casing layer in the integrated body by dip coating. The coating liquid composition was dried for one hour at normal temperature, and then the integrated body was treated for one hour in a hot air dryer at 60°C. The thickness of the adhesiveness enhancing layer thus obtained was about 1 µm,

| (Coating liquid 1 for formation of adhesiveness enhancing layer) | |
|---|---|
| • Liquid state epoxy resin (trade name: EXA-4850-150, manufactured by DIC Corp.) | 10 g |
| • Amidoamine-based curing agent (trade name: GENAMID 250, manufactured by Cognis GmbH) | 2.2 g |
| • Methyl ethyl ketone (manufactured by Wako Pure Chemical Industries, Ltd.) | 90 ml |

### (Formation of overcoat layer)

Subsequently, a coating liquid for overcoat having the following composition was prepared by dissolving the following soft fluororesin in a solvent.

| (Coating liquid for formation of overcoat layer) | |
|---|---|
| • Soft fluororesin (trade name: CEFRAL SOFT G-150, manufactured by Central Glass Co., Ltd.) (in Table 1, indicated as "soft fluororesin") | 10 g |
| • Dimethylformamide | 30 g |
| • Methyl ethyl ketone | 90 g |

The laminate obtained by laminating a casing layer 36 and an adhesiveness enhancing layer 38 on a metallic core material was immersed in this solution and was pulled out at a speed of 1 mm/sec to perform coating, and after the coating, the integrated body was heated and dried for 20 minutes in a hot air dryer at 120°C, to thereby providing an overcoat layer having a thickness of 5 µm. Thus, a flexible tube (A) of Example 1 according to the invention was produced.

The Shore hardness of the overcoat layer was measured, and the value was Shore D:50.

### [Example 2]

A flexible tube (B) of Example 2 was produced in the same manner as in Example 1, except that the adhesiveness enhancing layer was formed by performing coating using a coating liquid composition for the formation of an adhesiveness enhancing layer having the following composition in place of the composition used in Example 1 for the formation of an adhesiveness enhancing layer, at a heat treatment temperature of 120° for one hour.

| (Coating liquid for formation of adhesiveness enhancing layer 2) | |
|---|---|
| • Liquid state epoxy resin (trade name: EPICLON 840, manufactured by DIC Corp.) | 10 g |
| • Curing agent (trade name: JEFFAMINE D400, manufactured by manufactured by Huntsman International LLC) | 6.4 g |
| • Methyl ethyl ketone | 10 ml |
| • Cyclohexanone | 80 ml |

### [Comparative Example 1]

A flexible tube (C) of Comparative Example 1 was produced in the same manner as in Example 1, except that NEOPAINT FRONTOP #9200 (trade name, manufactured by Asia Industry Co., Ltd.; in Table 1, indicated as "reactive curing type fluororesin"), which is a reactive curing type fluorine coating agent, was used in place of the soft fluororesin, CEFRAL SOFT G-150, which was used in the formation of the overcoat layer.

### [Comparative Example 2]

A flexible tube (D) of Comparative Example 2 was produced in the same manner as in Example 1, except that the adhesiveness enhancing layer was formed using HARDLEN (trade name, manufactured by Toyo Kasei Kogyo Co., Ltd.), which is a chlorinated polyolefin, was used in place of the liquid state epoxy resin (trade name: EXA-4510-150, manufactured by DIC Corp.) and the amidoamine-based curing agent (trade name: GENAMID 250, manufactured by Cognis GmbH) used in the formation of the adhesiveness enhancing layer.

The respective flexible tubes produced were subjected to the following evaluation.

### (1) Autoclave treatment durability test

A durability test was carried out by placing the obtained flexible tubes in a small-sized gravity type autoclave apparatus, and leaving the flexible tubes to stand in an atmosphere of steam at 135°C for 2 weeks..

### (1-1) Change in external appearance

After the flexible tubes were taken out from the autoclave, an observation of the external appearance was made by visual inspection to examine the presence or absence of any change in the state such as cracking and peeling in the casing layer, overcoat layer and the like. Thus, the change in the external appearance was judged according to the following criteria.

### Evaluation criteria

A: No change in the external appearance is recognized.
B: Slight cracking and/or peeling is recognized.
C: Cracking and/or peeling is recognized over the entirety of the external appearance.

### (1-2) Bending durability test

Various samples having a length of 10 cm, which were separately produced, were left to stand in the autoclave in the same manner as in the observation of the external appearance, and were subsequently taken out and subjected to a repeated 90° bending test for 100 rounds using a testing machine. Then, an examination was conducted to see any change in the external appearance between the initial appearance and the appearance after the test.

### Evaluation criteria

A: No change in the external appearance was recognized.
B: Slight cracking and/or peeling was recognized.
C: Cracking and/or peeling is recognized over the entirety of the external appearance.

### (2) Hydrogen peroxide treatment durability test

The flexible tubes thus obtained were left to stand for two weeks in a 30% aqueous hydrogen peroxide solution maintained at 45°C, and thus a durability test was carried out.

### (2-1) Change in external appearance

After the flexible tubes were taken out from the aqueous hydrogen peroxide solution, an observation of the external appearance was made by visual inspection to examine the presence or absence of any change in the state such as cracking and peeling in the casing layer, overcoat layer and the like. Thus, the change in the external appearance was judged according to the following criteria.

### Evaluation criteria

A: No change in the external appearance is recognized.
B: Slight cracking and/or peeling is recognized.
C: Cracking and/or peeling is recognized over the entirety of the external appearance.

### (2-2) Bending durability test

Various samples having a length of 10 cm, which were separately produced, were immersed in an aqueous hydrogen peroxide solution in the same manner as in the observation of the external appearance, and were subsequently taken out and subjected to a repeated 90° bending test for 100 rounds using a testing machine. Then, an examination was conducted to see any change in the external appearance between the initial appearance and the appearance after the test.

### Evaluation criteria

A: No change in the external appearance was recognized.
B: Slight cracking and/or peeling was recognized.
C: Cracking and/or peeling is recognized over the entirety of the external appearance.

**[Table 1]**

| | Adhesiveness enhancing layer | Overcoat layer | Resistance to autoclaving | | Resistance to hydrogen peroxide | |
|---|---|---|---|---|---|---|
| | | Material | Appearance evaluation | Bending test | Appearance evaluation | Bending test |
| Example 1 | Soft epoxy resin | Particular fluororesin* 1 | A | A | A | A |
| Example 2 | Soft epoxy resin | Particular fluororesin * 1 | A | A | A | A |
| Comparative Example 1 | Soft epoxy resin | Reactive curing type fluororesin*2 | C | - | B | C |
| Comparative Example 2 | Chlorinated polyolefin | Particular fluororesin*1 | A | C | B | C |

| | | | | | | |
|---|---|---|---|---|---|---|
| *1 CEFRAL SOFT G-150 (trade name, manufactured by Central Glass Co., Ltd.) *2 NEOPAINT FRONTOP 9200 (trade name, manufactured by Asia Industry Co., Ltd.) | | | | | | |

As seen from the results of Table 1, all of the flexible tubes of the invention were not recognized to have deterioration in the external appearance after the autoclave treatment as well as the hydrogen peroxide treatment, and had excellent bending durability.

On the other hand, the flexible tube of Comparative Example 1 having an overcoat layer formed from a reactive curing type fluorine coating agent, which did not contain the vinylidene fluoride unit, exhibited noticeable deterioration in the external appearance such as peeling and deterioration of the overcoat layer due to the autoclave treatment, and did not exhibit bending durability. The flexible tube of Comparative Example 2 having an adhesiveness enhancing layer formed using a chlorinated polyolefin, was not recognized to have deterioration in the external appearance in the overcoat layer due to the autoclave treatment, but had poor bending durability. Furthermore, all of the flexible tubes were recognized to have partial peeling in the overcoat layer after the hydrogen peroxide treatment, and the peeling became more noticeable in the bending durability test.

According to the invention, there is provided a flexible tube for medical endoscopes which has damage or deterioration in the casing suppressed even when subjected to an autoclave sterilization treatment and a hydrogen peroxide plasma sterilization treatment, maintains necessary flexibility and protective property, and has excellent durability.

Furthermore, according to the invention, there is provided a method for producing, with high productivity, a flexible tube for medical endoscopes which has damage or deterioration in the casing suppressed even when subjected to an autoclave sterilization treatment and a hydrogen peroxide plasma sterilization treatment, maintains necessary flexibility and protective property, and has excellent durability.

## Claims

1. An endoscope (10) comprising an insertion section (12) which includes a flexible tube (12A), wherein the flexible tube comprises:
a metallic core material (32, 34) which has a helical tube (32) formed by winding a band-shaped member in a helical form, and a reticulated tube (34) formed along an outer periphery of the helical tube (32);
a casing layer (36) which is formed on a surface of the metallic core material (32, 34), wherein the casing layer (36) has an inner surface adhering to the surface of the metallic core material (32, 34) only at the area in contact with the reticulated tube to obtain an integrated body, and further wherein the casing layer (36) has a surface at an outer periphery thereof, that is subjected to an atmospheric pressure plasma treatment, and contains an olefin-based elastomer or a styrene-based elastomer;
an adhesiveness enhancing layer (38) which is formed along an outer periphery of the casing layer (36); and
an overcoat layer (40) which is formed along an outer periphery of the adhesiveness enhancing layer (38) and contains a fluororesin,
**characterized in that**
the adhesiveness enhancing layer (38) contains a flexible epoxy resin;
the fluororesin is a solvent-soluble fluororesin having a vinylidene fluoride unit as a polymerization component at a proportion of from 40 % by mole to 90 % by mole.

2. The endoscope (10) of claim 1, wherein the flexible epoxy resin is a reaction product between a curing agent having an amino group and an epoxy resin main agent selected from butanediol diglycidyl ether, a reaction product of polypropylene glycol and epichlorohydrin, or a bisphenol epoxy compound.

3. A method for producing a flexible tube (12A) of an insertion section (12) of an endoscope (10) as claimed in claim 1, the method comprising:
coating an olefin-based elastomer or a styrene-based elastomer on a surface of a metallic core material (32, 34) having a helical tube (32) formed by winding a band-shaped member in a helical form and a reticulated tube (34) formed along an outer periphery of the helical tube, and thereby forming a casing layer (36) on the metallic core material, wherein an inner surface of the casing layer (36) is adhered to the surface of the metallic core material (32,34) only at the area in contact with the reticulated tube to obtain an integrated body;
subjecting a surface at an outer periphery of the casing layer (36) to an atmospheric pressure plasma treatment;
applying a coating liquid composition along an outer periphery of the atmospheric pressure plasma-treated casing layer (36), and heating and drying the coating liquid composition, to form an adhesiveness enhancing layer (38); and
applying a coating liquid containing a fluororesin onto a surface of the adhesiveness enhancing layer (38), and drying and curing the coating liquid to form an overcoat layer (40),
**characterized in that**
the plasma treatment is performed to adjust a water contact angle at the surface of the casing layer (36) to a value of from 15° to 60°;
the coating liquid composition for forming the adhesiveness enhancing layer (38) contains a flexible epoxy resin;
the fluororesin is a solvent-soluble fluororesin having a vinylidene fluoride unit as a polymerization component at a proportion of from 40 % by mole to 90 % by mole.

## Patentansprüche

1. Endoskop (10) aufweisend einen Einführungsbereich (12), der einen flexiblen Schlauch (12A) besitzt, wobei der flexible Schlauch aufweist:
ein metallisches Kernmaterial (32, 34) mit einem Spiralschlauch (32), der ausgebildet ist durch Wickeln eines bandförmigen Elements in eine spiralige Form, und mit einem netzartigen Schlauch (34), der entlang eines Außenumfangs des Spiralschlauchs (32) ausgebildet ist;
eine Hüllenschicht (36), die auf einer Oberfläche des metallischen Kernmaterials (32, 34) ausgebildet ist, wobei die Hüllenschicht (36) eine Innenoberfläche hat, die an der Oberfläche des metallischen Kernmaterials (32, 34) nur an der Fläche, die mit dem netzartigen Schlauch in Kontakt ist, haftet, um einen einstückigen Körper zu erhalten, und wobei die Hüllenschicht (36) außerdem an ihrem Außenumfang eine Oberfläche hat, die einer Plasmabehandlung bei Atmosphärendruck unterzogen wurde und ein Elastomer auf Olefinbasis oder ein Elastomer auf Styrolbasis enthält;
eine haftungsverstärkende Schicht (38), die entlang eines Außenumfangs der Hüllenschicht (36) ausgebildet ist; und
eine Überzugsschicht (40), die entlang eines Außenumfangs der haftungsverstärkenden Schicht (38) ausgebildet ist und ein Fluorharz enthält,
**dadurch gekennzeichnet, dass**
die haftungsverstärkende Schicht (38) ein flexibles Epoxyharz enthält;
das Fluorharz ein in Lösungsmitteln lösliches Fluorharz mit einer Vinylidenfluorid-Einheit als eine Polymerisationskomponente in einem Anteil von 40 Mol-% bis 90 Mol-% ist.

2. Endoskop (10) nach Anspruch 1, wobei das flexible Epoxyharz ein Reaktionsprodukt zwischen einem Härter mit einer Aminogruppe und einem Epoxyharz-Hauptreaktionsmittel, das ausgewählt ist aus Butandiol-diglycidylether, einem Reaktionsprodukt von Polypropylen-glycol und Epichlorhydrin oder einer Bisphenolepoxy-Verbindung, ist.

3. Verfahren zur Herstellung eines flexiblen Schlauchs (12A) eines Einführungsbereichs (12) eines Endoskops (10), wie in Anspruch 1 beansprucht, wobei das Verfahren aufweist:
Auftragen eines Elastomers auf Olefinbasis oder eines Elastomers auf Styrolbasis auf eine Oberfläche eines metallischen Kernmaterials (32, 34) mit einem Spiralschlaucht (32), der ausgebildet ist durch Wickeln eines bandförmigen Elements in eine spiralige Form, und mit einem netzartigen Schlauch (34), der entlang eines Außenumfangs des Spiralschlauchs ausgebildet ist, und dadurch Ausbilden einer Hüllenschicht (36) auf dem metallischen Kernmaterial, wobei eine Innenoberfläche der Hüllenschicht (36) an der Oberfläche des metallischen Kernmaterials (32, 34) nur an der Fläche, die mit dem netzartigen Schlauch in Kontakt ist, haftet, um einen einstückigen Körper zu erhalten;
Unterziehen einer Oberfläche an einem Außenumfang der Hüllenschicht (36) einer Plasmabehandlung bei Atmosphärendruck;
Aufbringen einer Beschichtungsflüssigkeit-Zusammensetzung entlang eines Außenumfangs der bei Atmosphärendruck plasmabehandelten Hüllenschicht (36), und Erhitzen und Trocknen der Beschichtungsflüssigkeit-Zusammensetzung, um eine haftungsverstärkende Schicht (38) auszubilden; und
Aufbringen einer Beschichtungsflüssigkeit, die ein Fluorharz enthält, auf eine Oberfläche der haftungsverstärkenden Schicht (38), und Trocknen und Härten der Beschichtungsflüssigkeit, um eine Überzugsschicht (40) auszubilden,
**dadurch gekennzeichnet, dass**
die Plasmabehandlung durchgeführt wird, um einen Wasser-Kontaktwinkel an der Oberfläche der Hüllenschicht (36) auf einen Wert von 15° bis 60° einzustellen;
die Beschichtungsflüssigkeit-Zusammensetzung zur Ausbildung der haftungsverstärkenden Schicht (38) ein flexibles Epoxyharz enthält;
das Fluorharz ein in Lösungsmitteln lösliches Fluorharz mit einer Vinylidenfluorid-Einheit als eine Polymerisationskomponente in einem Anteil von 40 Mol-% bis 90 Mol-% ist.

## Revendications

1. Endoscope (10) comprenant une section d'introduction (12), laquelle inclut un tube flexible (12A), dans lequel le tube flexible comprend :
un matériau de coeur métallique (32, 34), lequel présente un tube hélicoïdal (32) formé en enroulant un élément en forme de bande sous une forme hélicoïdale, et un tube réticulé (34) formé le long d'une périphérie extérieure du tube hélicoïdal (32) ;
une couche enveloppante (36), laquelle est formée sur une surface du matériau de coeur métallique (32, 34), dans lequel la couche enveloppante (36) présente une couche intérieure adhérant à la surface du matériau de coeur métallique (32, 34) uniquement sur la zone en contact avec le tube réticulé afin d'obtenir un corps intégré, et en outre, dans lequel la couche enveloppante (36) présente une surface sur la périphérie extérieure, laquelle est soumise à un traitement au plasma à la pression atmosphérique, et contient un élastomère à base d'oléfine ou un élastomère à base de styrène ;
une couche améliorant l'adhésivité (38), laquelle est formée le long d'une périphérie extérieure de la couche enveloppante (36), et
une couche de surenrobage (40), laquelle est formée le long d'une périphérie extérieure de la couche améliorant l'adhésivité (38) et contient une fluororésine,
**caractérisé en ce que**
la couche améliorant l'adhésivité (38) contient une résine époxy flexible, et
la fluororésine est une fluororésine soluble dans un solvant présentant une unité de fluorure de vinylidène comme composant de polymérisation en une proportion allant de 40 % en moles à 90 % en moles.

2. Endoscope (10) selon la revendication 1, dans lequel la résine époxy flexible est un produit de réaction entre un agent de cuisson présentant un groupe amino et un agent principal de résine époxy sélectionné parmi l'éther de butanediol diglycidique, un produit de réaction de polypropylèneglycol et d'épichlorohydrine, ou un composé époxy de bisphénol.

3. Procédé destiné à produire un tube flexible (12A) d'une section d'introduction (12) d'un endoscope (10) selon la revendication 1, le procédé comprenant les étapes consistant à :
enrober avec un élastomère à base d'oléfine ou un élastomère à base de styrène une surface d'un matériau de coeur métallique (32, 34) présentant un tube hélicoïdal (32) formé en enroulant un élément en forme de bande sous une forme hélicoïdale et un tube réticulé (34) formé le long d'une périphérie extérieure du tube hélicoïdal, et en formant ainsi une couche enveloppante (36) sur le matériau de coeur métallique, dans lequel une couche intérieure de la couche enveloppante (36) adhère à la surface du matériau de coeur métallique (32, 34) uniquement sur la zone en contact avec le tube réticulé afin d'obtenir un corps intégré ;
soumettre une surface sur une périphérie extérieure de la couche enveloppante (36) à un traitement au plasma à la pression atmosphérique, et
appliquer une composition liquide d'enrobage le long d'une périphérie extérieure de la couche enveloppante (36) traitée au plasma à la pression atmosphérique, et chauffer et sécher la composition liquide d'enrobage afin de former la couche améliorant l'adhésivité (38),
appliquer un liquide d'enrobage contenant une fluororésine sur une surface de la couche améliorant l'adhésivité (38), puis sécher et cuire le liquide d'enrobage afin de former une couche de surenrobage (40),
**caractérisé en ce que**
le traitement au plasma est réalisé afin de régler un angle de contact avec l'eau à la surface de la couche enveloppante (36) sur une valeur allant de 15° à 60° ;
la composition de liquide d'enrobage pour former la couche améliorant l'adhésivité (38) contient une résine époxy flexible, et
la fluororésine est une fluororésine soluble dans un solvant présentant une unité de fluorure de vinylidène comme composant de polymérisation en une proportion allant de 40 % en moles à 90 % en moles.
